(19) Europäisches Patentamt
European Patent Office
Office européen des brevets

(11) EP 3 570 813 B1

(12) **EUROPEAN PATENT SPECIFICATION**

(45) Date of publication and mention of the grant of the patent:
**21.10.2020 Bulletin 2020/43**

(21) Application number: **18700141.7**

(22) Date of filing: **09.01.2018**

(51) Int Cl.:
*A61K 8/46* (2006.01) *A61K 8/49* (2006.01)
*A61Q 5/00* (2006.01) *A61Q 5/02* (2006.01)

(86) International application number:
**PCT/EP2018/050423**

(87) International publication number:
**WO 2018/134080 (26.07.2018 Gazette 2018/30)**

(54) **HAIR CARE COMPOSITION**

HAARPFLEGEZUSAMMENSETZUNG

COMPOSITION DE SOIN CAPILLAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.01.2017 PCT/CN2017/071774**
**16.03.2017 EP 17161265**

(43) Date of publication of application:
**27.11.2019 Bulletin 2019/48**

(73) Proprietors:
- **Unilever N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**
- **Unilever PLC**
**London Greater London EC4Y 0DY (GB)**
Designated Contracting States:
**CY GB IE MT**

(72) Inventors:
- **LI, Zhengrong**
**Shanghai 200335 (CN)**
- **LIU, Jian**
**Shanghai 200335 (CN)**
- **PAN, Xuemiao**
**Shanghai 200335 (CN)**
- **SUBRAMANIAN, Raghupathi**
**Shanghai 200335 (CN)**

(74) Representative: **Tansley, Sally Elizabeth**
**Unilever N.V.**
**Unilever Patent Group**
**Olivier van Noortlaan 120**
**3133 AT Vlaardingen (NL)**

(56) References cited:
**EP-A2- 0 338 850**
**WO-A1-2004/056329**
**WO-A1-2013/050241**
**US-A1- 2004 259 744**

- **DATABASE GNPD [Online] MINTEL; 31 January 2014 (2014-01-31), "LT High Performance Hair Stimulating Shampoo", XP002770577, Database accession no. 2190775**

EP 3 570 813 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

This invention relates to a personal care composition, more particularly to a shampoo composition, that is substantially free of sulphated surfactants. The judicious combination of specific surfactants in the present invention, as defined in the appended claims, is found to be capable of enhancing deposition of antimicrobial actives like azole compounds and concomitantly higher microbial kill.

### Background of the invention

Hair care compositions generally provide cleansing or conditioning benefits or a combination of the two. Such compositions typically comprise one or more cleansing surfactants which generally aid in cleaning the hair and the scalp free of undesirable soil, particles and fatty matter. Hair conditioners are another class of hair care compositions which are generally used on hair in the wet condition after the hair has been washed with a shampoo. Compositions which provide the dual benefits of shampooing and conditioning are also known.

Additionally, anti-dandruff benefit has been provided through hair care compositions. Dandruff is an issue that affects many people globally. The condition is manifested by the shedding of clumps of dead skin cells from the scalp. These are white in colour and provide an aesthetically displeasing appearance. A factor that contributes to dandruff are certain members of the Malassezia yeasts. To combat these, anti-dandruff products have included certain zinc salts which have anti-fungal activity, for example zinc pyrithione (ZPTO). Such a product has to perform as a hair cleansing shampoo, while mitigating the ill-effects of dandruff. An example of a known anti-dandruff shampoo comprises sodium lauryl ether sulfate (an ethoxylated anionic surfactant) in combination with an anti-dandruff agent. Typical anti-dandruff agents used in hair care are metal pyrithione e.g zinc pyrithione (ZPTO), octopirox (piroctone olamine), azole antimicrobials (e.g. climbazole), selenium sulfide and combinations thereof.

Hair shampoos generally comprise anionic surfactants and most shampoos presently comprise sulphated surfactants. (see e.g. WO 2013/050241 A1 or US 2004/259744 A1) The most popular surfactant combination (due to availability, cleaning efficacy and cost) is that of sodium lauryl ether sulphate (SLES) and Coco amido propyl betaine (CAPB). However sulphated surfactants like SLES have, over the years, acquired certain negative perceptions with some consumers. It is also expected that such compounds could face some issues in the future with some regulatory authorities. Therefore, there has been a trend to find alternative surfactant mixtures which would provide equivalent cleaning without compromise on the sensory. The present inventors arrived at the combination of sodium cocoyl isethionate and sodium lauryl sulphoacetate which has been used in certain premium shampoos in the past. They surprisingly found that this surfactant mixture (optionally and additionally with CAPB) is able to provide enhanced deposition of non-particulate antimicrobial actives like azoles (optionally with octopirox). The enhanced deposition is well correlated with enhanced antimicrobial activity in the present invention. It is observed that this enhanced deposition and antimicrobial kill is not observed for particulate active like ZPTO. Further, it is observed that cationic deposition polymers (like cationic guar or cationic cellulose) are not required for obtaining the enhanced deposition, although such polymers may be used.

In summary, the present inventors have found that the specific combination of sodium cocoyl isethionate and sodium lauryl sulphoacetate (optionally with CAPB) can enhance the deposition of azole (optionally with octopirox) antimicrobial actives.

### Summary of the invention

According to the first aspect of the present invention there is provided a hair care composition comprising from 5% to 15% by weight of sodium cocoyl isoethionate and from 0.1% to 5% by weight of sodium lauryl sulphoacetate; and

An antimicrobial active selected from an azole compound;
wherein the azole compound is climbazole or ketoconazole.

According to the second aspect of the present invention there is provided a method of depositing an antimicrobial active selected from an azole compound on to a desired scalp surface comprising the steps of applying a composition of the first aspect on to the desired surface followed by rinsing the surface with water.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or

options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. In other words, in specifying any ranges of values, any particular upper value can be associated with any particular lower value.

The disclosure of the invention, as found herein, is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) mutatis mutandis.

By 'A Hair Care Composition" as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or bar. Non-limiting examples of such compositions include leave-on hair lotions, creams, and wash-off shampoos, conditioners, shower gels, or toilet bar. The composition of the present invention is preferably a wash-off composition, especially preferred being a shampoo or a conditioner and most preferably a shampoo.

According to the first aspect of the present invention, there is provided a hair care composition comprising a combination of two surfactants which are sodium cocoyl isoethionate and sodium lauryl sulphoacetate.

The present invention relates to a hair composition comprising:
from 5% to 15% by weight of sodium cocoyl isoethionate and from 0.1% to 5% by weight of sodium lauryl sulphoacetate; and an antimicrobial active selected from an azole compound; wherein the azole compound is climbazole or ketoconazole.

Sodium Cocoyl Isethionate (CH3 (CH2)4-5CH2COOC2H4SO3Na) is a sodium salt ester of a fatty acid derived from coconut oil. It is used in cosmetics and personal care products as a surfactant and is often seen in hair care products like shampoos because of its ability to help water to mix with oil and dirt, allowing them to be more easily rinsed away. The chemical structure of Sodium Cocoyl Isethionate is as below:

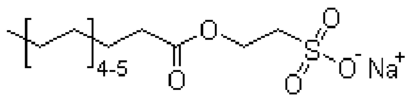

Sodium Lauryl Sulphoacetate (C14H27NaO5S) is derived from coconut and palm oils. It is a skin friendly cleanser that offers rich lather without the irritation potential, making it an excellent choice for soaks and facial cleansers and any other gentle cleansing systems. Sodium Lauryl Sulphoacetate has the chemical structure as below:

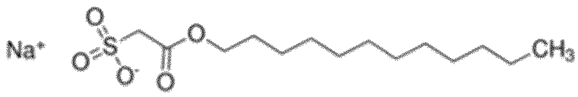

The composition as per the invention optionally and preferably additionally comprises a betaine surfactant. In a preferred embodiment, the composition comprises from 0.1 to 20 wt.%, preferably from 0.5 to 10 wt.%, more preferably from 1 to 8 wt.% of a betaine surfactant, preferably an alkyl amidopropyl betaine, for example cocamidopropyl betaine.

Shampoo compositions according to the invention may comprise additionally an alkyl sulphate and/or ethoxylated alkyl sulfate anionic surfactant, although this type of surfactant is not necessary to get the benefits of the invention. As another preferred aspect, these type of surfactants are substantially absent from the invention. By 'substantially absent' as per this invention, it is meant that these surfactants are present in less than 1%, preferably less than 0.1%, by the weight of the composition. Preferred alkyl sulfates are C8-18 alky sulfates, more preferably C12-18 alkyl sulfates, preferably in the form of a salt with a solubilising cation such as sodium, potassium, ammonium or substituted ammonium. Examples are sodium lauryl sulfate (SLS) or sodium dodecyl sulfate (SDS).

Preferred alkyl ether sulfates are those having the formula: RO (CH2CH2O) nSO3M; wherein R is an alkyl or alkenyl having from 8 to 18 (preferably 12 to 18) carbon atoms; n is a number having an average value of greater than

at least 0.5, preferably between 1 and 3, more preferably between 2 and 3; and M is a solubilising cation such as sodium, potassium, ammonium or substituted ammonium. An example is sodium lauryl ether sulfate (SLES).

Preferred ethoxylated alkyl sulfate anionic surfactant is sodium lauryl ether sulfate (SLES) having an average degree of ethoxylation of from 0.5 to 3, preferably 1 to 3.

If added, the total amount of additional anionic cleansing surfactant in shampoo compositions of the invention may generally range from 0.1 to 16 wt. %, preferably from 0.1 to 10 wt. %, calculated by total weight anionic cleansing surfactant based on the total weight of the composition.

Compositions of the invention comprise an antimicrobial agent preferably an anti-dandruff agent. Preferred antimicrobial agents include climbazole and ketoconazole. Preferably, the anti-microbial agent is in solution in the composition. The anti-microbial agent is therefore preferably soluble in the composition of the invention at 25 °C. The anti-microbial agent may be a single anti-microbial compound or a mixture of different anti-microbial compounds. Preferably, the anti-microbial agent is present in the composition in an amount of from 0.01 to 5% by weight, more preferably from 0.5 to 1% by weight.

The composition as per the invention preferably additionally comprises octopirox. The octopirox is preferably included in 0.01 to 5%. The presence of octopirox is believed to improve the deposition of conazole fungicide.

The composition also comprises a cationically modified polymer wherein the cationically modified polymer is cationically modified guar or cationally modified cellulose. Cationic guar deposition polymer is preferably guar hydroxypropyltrimonium chloride.

When conditioning benefits are to be delivered through the hair care composition of the invention the composition comprises a conditioning agent. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. An especially useful conditioning agent is a silicone compound, preferably a non-volatile silicone compound. Advantageously compositions herein may include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).

Amounts of the silicone in compositions, where present, may range from about 0.01 to about 10 wt.%, preferably from about 0.1 to about 8 wt.%, more preferably from about 0.3 to about 5wt.% by weight of the hair care compositions.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

According to another aspect of the invention there is provided a method of depositing an azole compound (or additionally octopirox) antimicrobial agent on to scalp comprising the steps of applying a composition of the invention on to the desired scalp surface followed by rinsing the surface with water. Such a method provides a uniform deposition profile. The method is preferably for non-therapeutic benefits.

The invention will now be illustrated with reference to the following non-limiting Examples.

## Examples

Example 1-4: Effect of combination of various surfactants and antimicrobial actives as per the invention on the deposition efficiency.

The following shampoo compositions were prepared.

Table-1

| Ingredient | Wt.% as active |
|---|---|
| Surfactant | As in Table -2 |
| Guar hydroxypropyltrimonium chloride | 0.2 |
| Propylene glycol | 1 |
| Antimicrobial actives | As in Table -2 |

(continued)

| Ingredient | Wt.% as active |
|---|---|
| Sodium salicylate | 0.35 |
| Sodium hydroxide | 0.25 |
| Carbopol 980 | 0.4 |
| Phenoxyethanol | 0.5 |
| Ethylene glycol distearate (EGDS) | 0.4 |
| Water | To 100 |

The antimicrobial active deposition and antimicrobial reduction efficacy of the various compositions were measured using an in vitro model and an anti-malassezia assay as given below.

In vitro model

About 0.2 grams of the shampoo composition was taken on artificial skin (VITRO-SKIN from IMS testing group). This was diluted with 1.8 ml water and rubbed with a plastic rod for 30 seconds. The surface was then rinsed two times with water, first time with 4 ml water for 30 seconds and then again with 4 ml water for 30 seconds. The ZPTO or Climbazole deposited on the skin was then measured using HPLC method. The average deposition (of five such experiments) and standard deviation is given in the Table-2 below.

Anti-malassezia assay

Halics was initially inoculated in Pityrosporum Broth and then transferred into agar slurry, to bring it to a final concentration of approximately 2 to $6x10^5$ cells/ml. Vitro-Skin™ was sandwiched in a plastic ring support, with its rough topography facing up. After treating with 0.2 g of shampoo and rinsing off twice with 1.8 ml water, the Vitro-Skin™ ring was placed onto a Modified Dixon Agar plate and 0.2 ml sodium chloride solution with Halics which was gently pipetted onto the rough skin surface. Following incubation for 24 hours, the Vitro-Skin™ was placed in a vial containing 10 ml Butterfield's phosphate buffer and then vortexed and ultrasonically treated. 20 $\mu$l of $10^0$ to $10^{-3}$ dilutions of the above solution was plated onto Modified Dixon Agar plates, and incubated for another 3 to 4 days. The number of colonies on each plate was then counted, and the final numbers were determined by multiplying by the appropriate dilution. The log reduction of each sample was calculated as follows and averaged from three replicates.

$$\text{Log reduction} = \text{Log}_{10}\text{CFU}_{(\text{Sample})} - \text{Log}_{10}\text{CFU}_{(\text{water control})}$$

The various shampoo compositions prepared above, had the surfactant and antimicrobial active specification as given in the Table-2 below.
The samples along with the data on the log kill is shown in Table-2 below.

Table-2

| Example Number | Surfactant combination (Conc. in wt. %) | Antimicrobial a ctive type (wt. %) | Antimicrobial active deposition, $\mu$g/plate | Std. dev | Log reduction (cfu/ml) | Std. dev |
|---|---|---|---|---|---|---|
| 1 | 8.4SCI/2.84SLAS | 0.5%Climbazole | 8.98 | 1.01 | -1.10 | 0.13 |
| A | 14SLES/1.6CAPB | 0.5%Climbazole | 3.92 | 0.22 | -0.32 | 0.14 |
| 2 | 8.4SCI/2.84SLAS/ 2.1CAPB | 0.5%Climbazole | 10.92 | 0.38 | -1.69 | 0.22 |
| 3 | 8.4SCI/2.84SLAS/ 2.1CAPB | 1%Climbazole | 12.01 | 1.36 | -2.01 | 0.09 |
| 4 | 8.4SCI/2.84SLAS/ 2.1CAPB | 5%Climbazole | 42.17 | 4.34 | -1.93 | 0.06 |

(continued)

| Example Number | Surfactant combination (Conc. in wt. %) | Antimicrobial a ctive type (wt. %) | Antimicrobial active deposition, μg/plate | Std. dev | Log reduction (cfu/ml) | Std. dev |
|---|---|---|---|---|---|---|
| B | 14SLES/1.6CAPB | 1% ZPTO | 1.12 | 0.08 | -0.56 | 0.20 |
| C | 8.4SCI/2.84SLAS/ 2.1CAPB | 1% ZPTO | 0.39 | 0.29 | -0.04 | 0.05 |
| Sodium Cocoyl Isethionate (SCI); Sodium Lauryl Sulphoacetate (SLAS); Coco amidopropyl betaine (CAPB); | | | | | | |

The data in Table-2 above indicates that compositions as per the invention (Examples 1-2) provide for better climbazole deposition and log reduction compared to the examples outside the invention (Example A) . These efficacies increase with conc. level of climbazole increasing in SCI/SLAS/CAPB system (Examples 2-4). However, it is observed that this enhanced deposition and antimicrobial kill is not observed for particulate active like ZPTO (Example B, C).

Example 3, 5-6: Effect of silicone oil and cationic guar as per the invention on the deposition efficiency and antimicrobial efficacy in the new surfactant system.

The following shampoo compositions was prepared.

Table-3

| Ingredient | Wt.% as active |
|---|---|
| Sodium Cocoyl Isethionate (SCI) | 8.4 |
| Sodium Lauryl Sulphoacetate (SLAS) | 2.84 |
| Coco amidopropyl betaine (CAPB) | 2.1 |
| Cationic guar | As in Table -4 |
| Silicone oil | As in Table -4 |
| Propylene glycol | 1 |
| Climbazole | 0.5 |
| Sodium salicylate | 0.35 |
| Sodium hydroxide | 0.25 |
| Carbopol 980 | 0.4 |
| Phenoxyethanol | 0.5 |
| Ethylene glycol distearate (EGDS) | 0.4 |
| Water | To 100 |

The various shampoo compositions prepared above, had the silicone oil and cationic guar specification as given in the Table-4 below. The antimicrobial agent deposition and antimicrobial reduction efficacy of the various compositions were measured using an invitro model and an anti-malassezia assay as described herein above.

Table-4

| Example Number | Silicone oil*, wt.% | Cationic guar**, wt.% | Climbazole deposition, μg/plate | Std. dev | Log reduction (cfu/ml) | Std. dev |
|---|---|---|---|---|---|---|
| 3 | 0 | 0.2% | 6.93 | 0.38 | -1.6945 | 0.22 |
| 5 | 0 | 0 | 7.19 | 1.21 | -1.5763 | 0.20 |
| 6 | 2% | 0.2% | 6.76 | 0.4 | -1.3871 | 0.11 |
| *Silicone oil used was Dimethiconol obtained from Dow Corning<br>**Cationic guar used was Guar Hydroxypropyltrimonium chloride obtained from Ashland | | | | | | |

The data in Table-4 above indicates that the addition of silicone oil or cationic guar (Examples 3, 5, 6) is not required to get enhanced climbazole deposition and log reduction efficacy.

## Claims

1. A hair care composition comprising:

   (i) from 5% to 15% by weight of sodium cocoyl isoethionate and from 0.1 % to 5% by weight of sodium lauryl sulphoacetate; and
   (ii) An antimicrobial active selected from an azole compound;

   wherein the azole compound is climbazole or ketoconazole.

2. A composition as claimed in claim 1 comprising 0.01 to 5% of the azole compound.

3. A composition as claimed in claim 1 or claim 2 additionally comprising octopirox.

4. A composition as claimed in claim 3 comprising 0.01% to 5% of the octopirox.

5. A composition as claimed in any one of the preceding claims additionally comprising a betaine surfactant.

6. A composition as claimed in claim 5 wherein the betaine surfactant is coco amido propyl betaine.

7. A composition as claimed in any one of the preceding claims additionally comprising an alkyl sulphate and/or ethoxylated alkyl sulfate anionic surfactant.

8. A composition as claimed in any one of the preceding claims comprising a cationically modified polymer.

9. A composition as claimed in claim 8 wherein the cationically modified polymer is cationically modified guar or cationically modified cellulose.

10. A composition as claimed in claim 9 wherein the cationically modified guar is guar hydroxypropyl trimonium chloride.

11. A composition as claimed in any one of the preceding claims wherein the composition is a shampoo.

12. A composition as claimed in any one of the preceding claims for use in a method of treating dandruff by depositing antimicrobial actives selected from an azole compound on to a desired scalp surface comprising the steps of applying the composition on to the desired surface followed by rinsing the surface with water.

## Patentansprüche

1. Haarpflegezusammensetzung, umfassend:

   (i) 5 bis 15 Gewichts-% Natriumcocoylisoethionat und 0,1 bis 5 Gewichts-% Natriumlaurylsulfoacetat; und
   (ii) einen antimikrobiellen Wirkstoff, ausgewählt aus einer Azolverbindung;

   wobei die Azolverbindung Climbazol oder Ketoconazol ist.

2. Zusammensetzung wie in Anspruch 1 beansprucht, umfassend 0,01 bis 5% der Azolverbindung.

3. Zusammensetzung wie in Anspruch 1 oder Anspruch 2 beansprucht, zusätzlich umfassend Octopirox.

4. Zusammensetzung wie in Anspruch 3 beansprucht, umfassend 0,01% bis 5% des Octopirox.

5. Zusammensetzung wie in irgendeinem der vorhergehenden Ansprüche beansprucht, zusätzlich umfassend ein Betaintensid.

6. Zusammensetzung wie in Anspruch 5 beansprucht, wobei das Betaintensid Cocoamidopropyl-Betain ist.

7. Zusammensetzung wie in irgendeinem der vorhergehenden Ansprüche beansprucht, zusätzlich umfassend ein Alkylsulfat und/oder anionisches ethoxyliertes Alkylsulfattensid.

8. Zusammensetzung wie in irgendeinem der vorhergehenden Ansprüche beansprucht, umfassend ein kationisch modifiziertes Polymer.

9. Zusammensetzung wie in Anspruch 8 beansprucht, wobei das kationisch modifizierte Polymer kationisch modifiziertes Guar oder kationisch modifizierte Cellulose ist.

10. Zusammensetzung wie in Anspruch 9 beansprucht, wobei das kationisch modifizierte Guar Guarhydroxypropyltrimoniumchlorid ist.

11. Zusammensetzung wie in irgendeinem der vorhergehenden Ansprüche beansprucht, wobei die Zusammensetzung ein Shampoo ist.

12. Zusammensetzung wie in irgendeinem der vorhergehenden Ansprüche beansprucht zur Verwendung in einem Verfahren zum Behandeln von Schuppen durch Abscheiden antimikrobieller Wirkstoffe, ausgewählt aus einer Azolverbindung, auf einer gewünschten Kopfhautoberfläche, umfassend die Schritte des Aufbringens der Zusammensetzung auf die gewünschte Oberfläche, gefolgt von Spülen der Oberfläche mit Wasser.

**Revendications**

1. Composition pour le soin des cheveux comprenant :

   (i) de 5 % à 15 % en masse de cocoylisoéthionate de sodium et de 0,1 % à 5 % en masse de laurylsulfoacétate de sodium ; et
   (ii) un actif antimicrobien choisi parmi un composé d'azole ;

   dans laquelle le composé d'azole est le climbazole ou kétoconazole.

2. Composition selon la revendication 1 comprenant de 0,01 à 5 % du composé d'azole.

3. Composition selon la revendication 1 ou revendication 2 comprenant de plus de l'octopirox.

4. Composition selon la revendication 3 comprenant de 0,01 % à 5 % de l'octopirox.

5. Composition selon l'une quelconque des revendications précédentes comprenant de plus un tensioactif de bétaïne.

6. Composition selon la revendication 5, dans laquelle le tensioactif de bétaïne est la cocoamidopropylbétaïne.

7. Composition selon l'une quelconque des revendications précédentes comprenant de plus un sulfate d'alkyle et/ou tensioactif anionique de sulfate d'alkyle éthoxylé.

8. Composition selon l'une quelconque des revendications précédentes comprenant un polymère cationiquement modifié.

9. Composition selon la revendication 8, dans laquelle le copolymère cationiquement modifié est un guar cationiquement modifié ou une cellulose cationiquement modifiée.

10. Composition selon la revendication 9, dans laquelle le guar cationiquement modifié est du chlorure de guar hydroxypropyltrimonium.

11. Composition selon l'une quelconque des revendications précédentes, dans laquelle la composition est un shampoing.

**12.** Composition selon l'une quelconque des revendications précédentes pour une utilisation dans un procédé de traitement de pellicules par dépôt d'actifs antimicrobiens choisis parmi un composé d'azole sur une surface de cuir chevelu souhaitée comprenant les étapes d'application de la composition sur la surface souhaitée suivie par le rinçage de la surface avec de l'eau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013050241 A1
- US 2004259744 A1